(19) Europäisches Patentamt
European Patent Office
Office européen des brevets

(11) EP 1 857 834 A2

(12) EUROPEAN PATENT APPLICATION

(43) Date of publication:
21.11.2007 Bulletin 2007/47

(51) Int Cl.:
G01S 15/89 (2006.01)   G01S 7/52 (2006.01)
G06T 17/00 (2006.01)   A61B 8/00 (2006.01)

(21) Application number: 07009599.7

(22) Date of filing: 14.05.2007

(84) Designated Contracting States:
AT BE BG CH CY CZ DE DK EE ES FI FR GB GR HU IE IS IT LI LT LU LV MC MT NL PL PT RO SE SI SK TR
Designated Extension States:
AL BA HR MK YU

(30) Priority: 16.05.2006  KR 20060043668

(71) Applicant: MEDISON CO., LTD.
Kangwon-do 250-870 (KR)

(72) Inventors:
• LEE, Seung Woo,
Discusser & Medison Building,
Seoul 135-280 (KR)

• KIM, Cheol An,
Discusser & Medison Building,
Seoul 135-280 (KR)
• SHIN, Seong Chul,
Discusser & Medison Building,
Seoul 135-280 (KR)

(74) Representative: Lorenz, Werner et al
Lorenz & Kollegen
Patent- und Rechtsanwaltskanzlei
Alte Ulmer Strasse 2
89522 Heidenheim (DE)

(54) **Ultrasound system for fusing an ultrasound image and an external medical image**

(57) There is provided an ultrasound system, which includes: an ultrasound diagnostic unit having a probe for transmitting an ultrasound beam to a target object and receiving ultrasound echo signals reflected from the target object to form ultrasound images; a position tracking unit for providing position information of the probe and ultrasound beam direction information; an external medical image signal providing unit for providing external medical image signals acquired from an external medical imaging device to form at least one external medical image; a user input unit for inputting position information of a lesion in the external medical image from a user; and an image processing unit for forming a fusion image of the ultrasound image and the external image based on the position information of the probe, the ultrasound beam direction information and the position information of the lesion in the external image.

FIG. 1

**Description**

The present application claims priority from Korean Patent Application No. 10-2006-43668 filed on May 16, 2006, the entire subject matter of which is incorporated herein by reference.

## BACKGROUND

### 1. Field

The present invention generally relates to ultrasound systems, and more particularly to an ultrasound system for fusing an ultrasound image and an external medical image acquired from an external medical imaging device.

### 2. Background

Surgical treatment using a medical needle such as ablator or biopsy has recently become popular due to relatively small incisions made in such a procedure. The surgical treatment is performed by inserting the medical needle into an internal region of a human body while referring to an internal image of the human body. Such surgical treatment, which is performed while observing internal organs of the human body by using a diagnostic imaging system, is referred to as an interventional treatment. The interventional treatment is performed by directing the medical needle to the lesion to be treated or examined through a skin with reference to images during the treatment. The images are acquired by employing a computerized tomography (CT) scanner generally used in a radiology department or a magnetic resonance imaging (MRI) system. Compared to a normal surgical treatment requiring relatively wide incisions to open the lesion, the interventional treatment has the advantages of low costs and obtaining effective operation results. This is because general anesthesia is not necessary for the interventional treatment and patients are subjected to less pain while benefiting from rapid recovery.

However, it is difficult to obtain such images in real time by using the CT scanner or the MRI system. Especially, when the interventional treatment is performed by using the CT scanner, both the patient and the operator are exposed to radiation for quite a long time. In contrast, when the interventional treatment is performed by using an ultrasound diagnostic system, the images can be obtained in real time while not affecting the human body. However, there is a problem in that it is difficult to accurately recognize the lesion in the ultrasound image obtained by using the ultrasound diagnostic system.

## BRIEF DESCRIPTION OF THE DRAWINGS

Arrangements and embodiments may be described in detail with reference to the following drawings in which like reference numerals refer to like elements and wherein:

FIG. 1 is a block diagram showing an ultrasound system constructed according to one embodiment of the present invention;

FIG. 2 is a block diagram showing a position tracking unit in accordance with one embodiment of the present invention;

FIG. 3 is a block diagram showing an image processing unit in accordance with one embodiment of the present invention;

FIG. 4 is a block diagram showing an image processing unit in accordance with one embodiment of the present invention;

FIG. 5 is a photograph showing an external image wherein a position marker attached to a lesion is displayed;

FIG. 6 is a schematic diagram showing an example of displaying ultrasound slice images and external slice images on a screen partitioned into two regions; and

FIG. 7 is a view showing an example of displaying a 3-dimensional image formed by reconstructing selected slice images at an analysis mode.

## DETAILED DESCRIPTION

A detailed description may be provided with reference to accompanying drawings. One of ordinary skill in the art may realize that the following description is illustrative only and is not in any way limiting. Other embodiments of the present invention may readily suggest themselves to such skilled persons having the benefit of this disclosure.

The present invention provides an ultrasound system for displaying a fusion image of an ultrasound image and an external medical image to more accurately observe a lesion. Hereinafter, one embodiment of the present invention will be described with reference to the accompanying drawings.

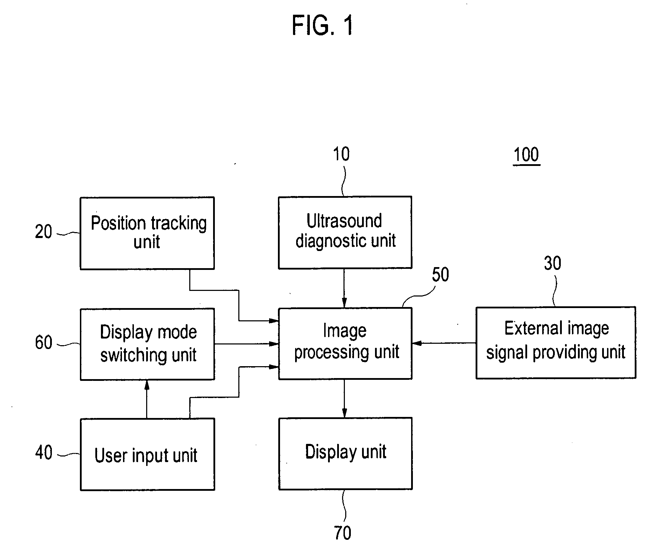

FIG. 1 is a block diagram showing an ultrasound system constructed according to one embodiment of the present invention. As shown in FIG. 1, the ultrasound system 100 includes an ultrasound diagnostic unit 10, a position tracking unit 20, an external image signal providing unit 30, a user input unit 40, an image processing unit 50, a display mode switching unit 60 and a display unit 70. The ultrasound diagnostic unit 10 includes an ultrasound image forming unit and a probe. The probe transmits ultrasound signals to a target object and receives ultrasound signals reflected from a target object in real time.

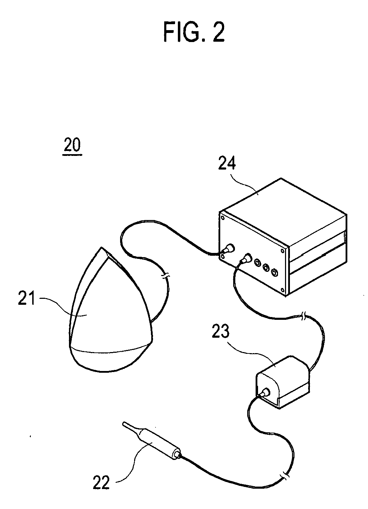

The position tracking unit 20 provides position information and ultrasound beam direction information of the probe, which is located at specific surface regions of a target object while the target object is scanned. Also, the position tracking unit 20 provides position information of specific regions (e.g., lesions) in external images acquired by an external imaging device such as a computer tomography (CT) scanner, a magnetic resonance imaging (MRI) system or the like. The position information of the lesions is obtained by using position information of the markers attached to the surface of the target object. As shown in FIG. 2, the position tracking unit 20 includes position markers (not denoted), a field generator 21, a position detector 22 and a position information generator 23. The position markers are attached to the surface of the target object to be displayed in the external images such as a CT image or an MRI image for indicating the positions of the lesions therein. The field generator 21 generates an electromagnetic field for tracking the position and the ultrasound beam direction of the probe. The position detector 22, which is mounted on a surface of the probe or built in the probe, generates a detection signal in response to the electromagnetic field generated from the field generator 21. The position information generator 23 generates position information and ultrasound beam direction information of the probe based on the detection signal received from the position detector 22. The position markers may be any type of substances, which are capable of being displayed in the CT image or the MRI image for indicating the positions of the lesions. The position detector 22 may be embodied with a coil sensor.

The external image signal providing unit 30 provides the external image signals acquired from the external image device to the ultrasound diagnostic unit 10. The external image signal may be provided from the CT scanner or the MRI system. The external image signals may be provided in a digital imaging communication format such as the digital imaging communication in medicine (DICOM) standard format.

The user input unit 40 receives position information of the lesion in the external image, a fusion condition of an ultrasound image and the external image, and a display mode switching request. The user input unit 40 may be a mouse, a keyboard, a track ball or the like. A method for designating the position of the lesion in the external image will be described later.

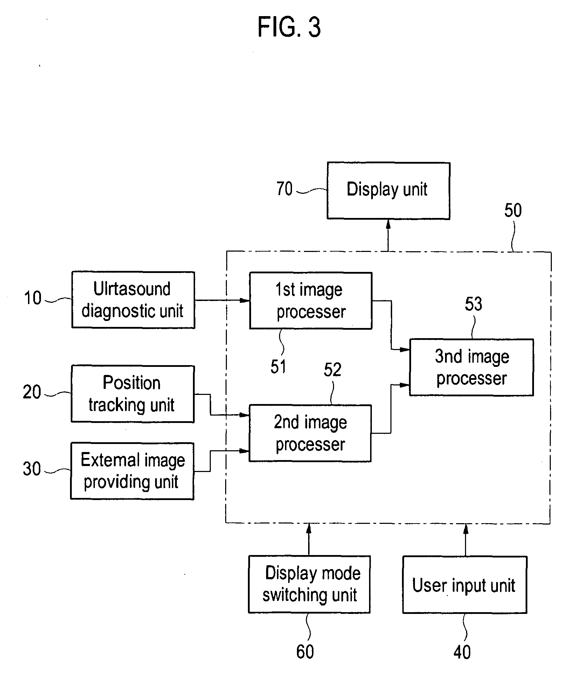

The image processing unit 50 includes first, second and third image processors 51, 52 and 53, as shown in FIG. 3. The first image processor 51 forms the ultrasound images based on the ultrasound echo signals inputted to the ultrasound diagnostic unit 10. The ultrasound images may include 2-dimensional ultrasound images, 3-dimensional ultrasound images and slice images. The second image processor 52 matches the coordinates of the external image with the coordinates representing probe positions based on the position information of the markers in the external image and the position information of the probe, which is located in each marker. The external image may be reconstructed to a 2-dimensional image, a 3-dimensional image or a slice image according to the coordinate matching result in the second image processor 52. The third image processor 53 fuses the ultrasound image and the reconstructed external image received from the first and second image processors 51 and 52, respectively.

The display mode switching unit 60 switches the display mode in response to the display mode switching request inputted from the user input unit 40. The display mode may include an ultrasound image display mode, an external image display mode, a fusion image display mode, a multi-slice image display mode of the ultrasound image and the external image and a volume analysis mode for rendering selected slices to form a 3-dimensional image and displaying the 3-dimensional image.

The display unit 70 displays at least one image of the ultrasound image, the external image and the fusion image under the control of the display mode switching unit 60. The display unit 70 may also simultaneously display at least two images of the ultrasound image, the external image and the fusion image.

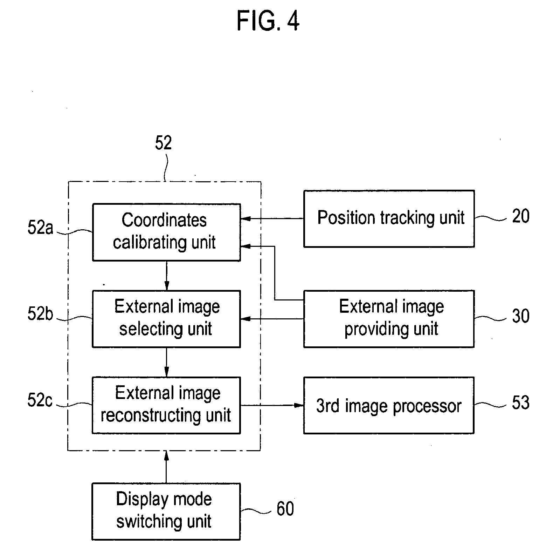

Hereinafter, an operation of the second image process 52 will be described in detail with reference to FIG. 4. The second image processor 52 includes a coordinate calibration unit 52a, an external image selection unit 52b and an external image reconstruction unit 52c.

The coordinate calibration unit 52a performs calibration upon origins in different coordinate systems including a coordinate system representing the external image such as the CT image or the MRI image and a coordinate system representing the position of the probe. That is, the coordinate calibration unit 52a calibrates the coordinates of the lesion in the external image to be matched with the coordinates representing the position of the probe. For this calibration, the coordinate calibration unit 52a generates the coordinates of the lesion in the ultrasound image based on the position information of the probe inputted from the position information generator 23. In this case, the probe is located in the maker attached to the surface of the target object for indicating the position of the lesion. The coordinate calibration unit 52a calibrates the coordinates of the lesion in the external image, which are inputted through the user input unit 40, based on the position coordinates of the probe located in the marker. In accordance with one embodiment of the present invention, the coordinates of the lesion may be calibrated by using a 4-point matching method.

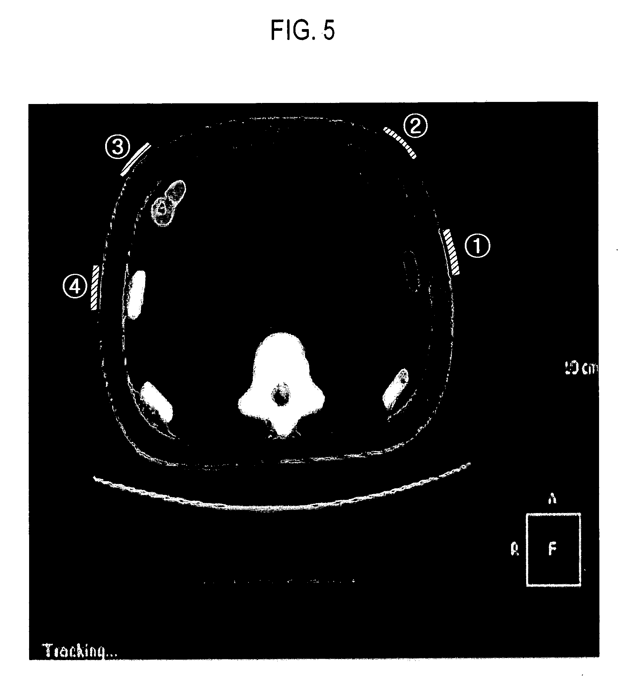

As shown in FIG. 5, for example, the four markers ①, ②, ③ and ④ attached to the surface of the target object for indicating the lesions are displayed on the external image. The user may select the external image, in which the markers are displayed, and then sequentially designate the markers in the selected external image through the user input unit 40. The user may designate the positions of the markers through a mouse click or the like. The coordinates corresponding to the positions of the markers in the external image are matched with the coordinates corresponding to the positions of the probe located in each marker, thereby calibrating the coordinates of the lesion position in the external image.

If the positions of the markers in the external image are expressed as position vectors g1, g2, g3 and g4, and the positions of the probe are expressed as position vectors v1, v2, v3 and v4, then the position vectors v1, v2, v3 and v4 may be considered as vectors obtained by applying a transform matrix M to the position vectors g1, g2, g3 and g4 as the following equation (1).

$$[v1\ v2\ v3\ v4] = M[g1\ g2\ g3\ g4] \tag{1}$$

The transform matrix M is defined as the following equation (2).

$$M = [v1\ v2\ v3\ v4][g1\ g2\ g3\ g4]^{-1} \tag{2}$$

As mentioned above, the coordinate calibration unit 52a applies the transform matrix M to the coordinates of the external image, thereby matching the coordinates of the external image with the coordinates of the ultrasound image. The external image selection unit 52b selects an external image based on the position information of the marker and the ultrasound beam direction information. That is, after matching the coordinates of the markers in the external image with the coordinates of the probe position, the external image selection unit 52b selects the external image that lies in the direction corresponding to the ultrasound beam direction. The external image reconstruction unit 52c reconstructs the selected external image based on the coordinate calibration result. Thereafter, the reconstructed image may be rendered.

The ultrasound image and the external image may be fused in a voxel unit. The third image processor 53 may perform a minimum value-based fusing process, a maximum value-based fusing process or a weighted value-based fusing process according to the fusion condition inputted through the user input unit 40. A fusion voxel value Vf defined by a voxel value Vmc of the external image and a voxel value Vus of the ultrasound image according to the minimum value-based fusing process, the maximum value-based fusing process and the weighted value-based fusing process may be represented as the following equations (3), (4) and (5), respectively.

$$V_f(x,y,z) = Min(V_{mc}(x,y,z), V_{us}(x,y,z)) \tag{3}$$

$$V_f(x,y,z) = Max(V_{mc}(x,y,z), V_{us}(x,y,z)) \tag{4}$$

$$V_f(x,y,z) = \alpha \times (V_{mc}(x,y,z), (1-\alpha) \times V_{us}(x,y,z)) \tag{5}$$

In the equation (5), $\alpha$ represents a weight value.

Hereinafter, an operation of the display mode change will be described in detail. If a selection request for selecting an ultrasound image display mode or an ultrasound image multi-slice mode is inputted through the user input unit 40, then the display mode switching unit 60 makes a 2-dimensional or 3-dimensional ultrasound image, or multi-slice ultrasound images to be transmitted from the first image processor 51 to the display unit 70. If a selection request for selecting an external image display mode or an external image multi-slice image display mode is inputted through the user input unit 40, then the display mode switching unit 60 makes an external image or the multi-slice external images to be transmitted from the second image processor 62 to the display unit 70. Further, if a selection request for selecting a fusion image display mode is inputted through the user input unit 40, then the display mode switching unit 60 makes a fusion image to be transmitted from the third image processor 53 to the display unit 70.

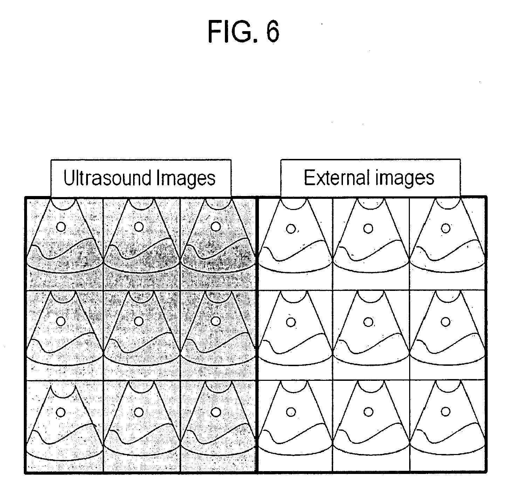

FIG. 6 is a schematic diagram showing an example of displaying ultrasound slice images and external slice images on a screen partitioned into two regions. If a selection request for selecting the multi-slice images of the ultrasound image and the external image is inputted through the user input 40, then the display unit 70 receives the ultrasound multi-slice images and external multi-slice images from the first and second image processors 51 and 52, respectively, under the control of the display mode switching unit 60. The display unit 70 displays the ultrasound multi-slice images and external multi-slice images on a screen partitioned into two regions as shown in FIG. 6.

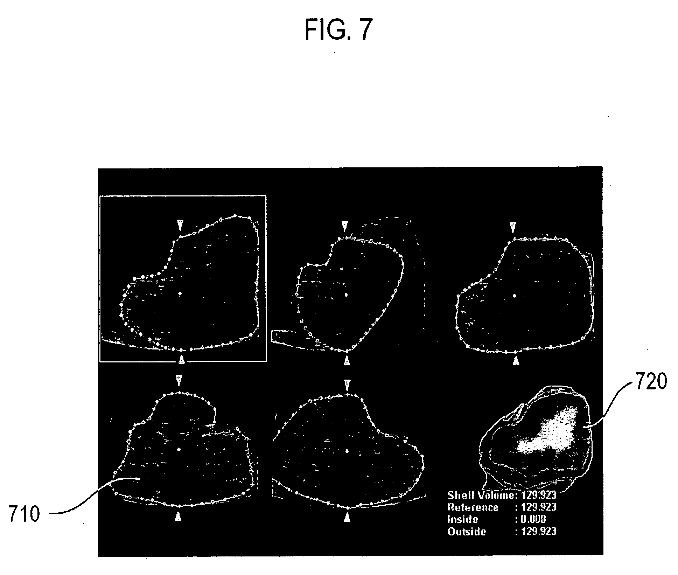

Further, in case the volume analysis mode is inputted through the user input 40, the selected slice images 710 are reconstructed, thereby forming a 3-dimensional image 720 as shown in FIG. 7. Whether the volume analysis mode is inputted is determined upon the user's selection of a specific slice image in the multi slice mode.

As mentioned above, since the fusion image of the ultrasound image and the external image is displayed in accordance with the present invention, the lesion in the target object can be more easily recognized. Therefore, it can provide convenience to an interventional ultrasound clinical application and reliability thereof can be improved.

An embodiment may be achieved in whole or in parts by the ultrasound system, including: an ultrasound diagnostic unit having a probe for transmitting ultrasound beam to a target object and receiving ultrasound echo signals reflected from the target object to form ultrasound images; a position tracking unit for providing position information of the probe and ultrasound beam direction information; an external medical image signal providing unit for providing external medical image signals acquired from an external medical imaging device to form at least one external medical image; a user input unit for inputting position information of a lesion in the external medical image from a user; and an image processing unit for forming a fusion image of the ultrasound image and the external image based on the position information of the probe, the ultrasound beam direction information and the position information of the lesion in the external image.

Any reference in this specification to "one embodiment," "an embodiment," "example embodiment," etc., means that a particular feature, structure or characteristic described in connection with the embodiment is included in at least one embodiment of the invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

**Claims**

1. An ultrasound system, comprising:

   an ultrasound diagnostic unit having a probe for transmitting an ultrasound beam to a target object and receiving ultrasound echo signals reflected from the target object to form ultrasound images;
   a position tracking unit for providing position information of the probe and ultrasound beam direction information;
   an external medical image signal providing unit for providing external medical image signals acquired from an external medical imaging device to form at least one external medical image;
   a user input unit for inputting position information of a lesion in the external medical image from a user; and
   an image processing unit for forming a fusion image of the ultrasound image and the external image based on the position information of the probe, the ultrasound beam direction information and the position information of the lesion in the external image.

2. The ultrasound system of Claim 1, wherein the position tracking unit includes:

a plurality of position markers attached to a surface of the target object over the lesion for indicating the position of the lesion in the external image;
a field generator for generating an electromagnetic field to track the position of the probe;
a detector mounted on or built in the probe for generating detection signals corresponding to the position of the probe and the ultrasound beam direction at each marker in response to the electromagnetic field; and
a position information generating unit for generating the position information of the probe and the ultrasound beam direction information based on the detection signal.

3. The ultrasound system of Claim 2, wherein the image processing unit includes:

a first image processor for forming the ultrasound images based on the ultrasound echo signals;
a second image processor for reconstructing the external images based on the position information of the probe, the ultrasound beam direction information and the position information of the lesion in the external image; and
a third image processor for fusing the ultrasound image and the external image received from the first image processor and the second image processor, respectively.

4. The ultrasound system of Claim 3, wherein the second image processor includes:

a coordinate calibration unit for generating coordinates representing the probe position based on the position information of the probe and calibrating coordinates of the lesion in the external image based on the coordinates representing the probe position;
an external image selection unit for selecting one of external medical images based on the coordinate calibration result and the ultrasound beam direction information; and
an external image reconstruction unit for reconstructing the selected external medical image.

5. The ultrasound system of Claim 1, wherein the external medical image device is a computer tomography scanner or a magnetic resonance imaging system.

6. The ultrasound system of Claim 5, wherein the ultrasound echo signals are inputted in real time.

7. The ultrasound system of Claim 1, wherein the user input unit receives fusion conditions of the ultrasound image and the external image, as well as a display mode switching request.

8. The ultrasound system of Claim 7, further comprising a display mode switching unit for switching a display mode in response to the display mode switching request.

# FIG. 1

```
                                              10              100

20 ──┤ Position tracking │      │ Ultrasound      │
       │      unit        │      │ diagnostic unit │
                                                          50              30

60 ──┤ Display mode     │ ──→  │ Image           │ ←── │ External image        │
       │ switching unit  │ ──→  │ processing unit │      │ signal providing unit │

40 ──┤ User input unit  │      │ Display unit    │

                                        70
```

# FIG. 2

# FIG. 3

70 —— Display unit

50

10 —— Ulrtasound diagnostic unit → 1st image processer

51

20 —— Position tracking unit

30 —— Extemal image providing unit → 2nd image processer

52

3nd image processer

53

Display mode switching unit

60

User input unit

40

# FIG. 4

```
                    52

   52a ┌─── Coordinates          ◄───── Position tracking unit ─── 20
        │    calibrating unit   ◄──┐
        │         │                │
        │         ▼                │
   52b ─┤    External image    ◄───┼── External image ─── 30
        │    selecting unit        │    providing unit
        │         │                │
        │         ▼                │
   52c ─┤    External image    ─────────► 3rd image processor ─── 53
        │    reconstructing unit
        └─────────▲─────────────

              Display mode        ─── 60
              switching unit
```

FIG. 5

# FIG. 6

# FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 10200643668